Europäisches Patentamt

European Patent Office

Office européen des brevets

(19)

(11) Veröffentlichungsnummer : **0 080 644**
**B1**

# (12) EUROPÄISCHE PATENTSCHRIFT

(45) Veröffentlichungstag der Patentschrift :
30.10.85

(21) Anmeldenummer : 82110548.3

(22) Anmeldetag : 16.11.82

(51) Int. Cl.⁴ : $C\ 07\ C143/66$

(54) Verfahren zur Herstellung von 1-Amino-2-naphthol-4-sulfonsäure (Amidolsäure).

(30) Priorität : 27.11.81 DE 3147152

(43) Veröffentlichungstag der Anmeldung :
08.06.83 Patentblatt 83/23

(45) Bekanntmachung des Hinweises auf die Patenterteilung : 30.10.85 Patentblatt 85/44

(84) Benannte Vertragsstaaten :
CH DE FR GB IT LI

(56) Entgegenhaltungen :
DE-C- 356 973
BERICHTE DER DEUTSCHEN CHEMISCHEN GESELL-
SCHAFT, Band 62, 1929, Seiten 68-80, Berlin, DE. N.N.
WOROSHTZOW et al.: "Über die Einwirkung von
Natriumbisulfit auf Nitroso-naphthole. (Studien in der
Naphthalin-Reihe, II)"
ORGANIC SYNTHESIS COLLECTIVE, Band 2, 1943,
Seiten 42-44 L.F. FIESER: "1-Amino-2-naphthol-4-sul-
fonic acid"

(73) Patentinhaber : BAYER AG
Konzernverwaltung RP Patentabteilung
D-5090 Leverkusen 1 Bayerwerk (DE)

(72) Erfinder : Hammerschmidt, Erich, Dr.
Schützenstrasse 25
D-5060 Bergisch-Gladbach 2 (DE)
Erfinder : Behre, Horst, Dr.
Zur alten Linde 12
D-5068 Odenthal (DE)
Erfinder : Blank, Heinz Ulrich, Dr.
Am Geusfelde 35
D-5068 Odenthal (DE)
Erfinder : Lindner, Otto, Dr.
Strässchen Siefen 31
D-5060 Bergisch-Gladbach 2 (DE)

EP 0 080 644 B1

**0 080 644**

**Beschreibung**

Die vorliegende Erfindung betrifft ein Verfahren zur Herstellung von 1-Amino-2-naphthol-4-sulfonsäure (Amidolsäure) durch Umsetzen von 3-Oxo-4-hydroxyimino-1,2,3,4-tetrahydronaphthalin-1-sulfonsäure mit Hydrogensulfit (Bisulfit) und einer Mineralsäure.

Amidolsäure ist ein wichtiges Zwischenprodukt zur Herstellung von Farbstoffen (Ullmanns Enzyklopädie der technischen Chemie, 4. Auflage, Band 17, Seite 100).

Ihre Herstellung ist beispielsweise in BIOS Final Report No. 986/I, Nr. 22, Seite 44-45 (vgl. auch Ullmann, loc. cit.) und in Organic Synthesis, Col. Vol. II, Seite 42 (1943) durch Umsetzung von nitrosiertem β-Naphthol mit Natriumhydrogensulfit und anschließende Ansäuerung mit Schwefelsäure beschrieben. Die hierbei zwischendurch erhaltenen wäßrigen Lösungen der 3-Oxo-4-hydroxyimino-1,2,3,4-tetrahydronaphthalin-1-sulfonsäure können auch durch Bisulfit-Addition an 1,2-Naphthochinon-1-oxim (1-Nitroso-2-naphthol) erhalten werden und dann in Gegenwart von mindestens äquimolaren Mengen Bisulfit angesäuert und erwärmt werden (Liebigs Ann. *638*, 76 (1960)). Die im erwähnten BIOS Final Report angegebene Vorschrift für die Herstellung von Amidolsäure hat aufgrund der Nachreaktionszeiten von 36 bis 40 Stunden und den sich daraus ergebenden niedrigen Raum/Zeit-Ausbeuten erhebliche Nachteile. Die in Organic Synthesis gemachten Ausbeuteangaben beziehen sich auf Trockenausbeuten, deren Gehalte an Amidolsäure ungenannt bleiben. Die Ausbeuten an 100 %iger Amidolsäure liegen daher unter den angegebenen.

Es wurde nun ein Verfahren zur Herstellung von 1-Amino-2-naphthol-4-sulfonsäure (Amidolsäure) durch Ansäuern von 3-Oxo-4-hydroxyimino-1,2,3,4-tetrahydronapthalin-1-sulfonsäure oder ihres Alkalisalzes in Gegenwart von Hydrogensulfit auf pH-Werte von etwa 1,5 bis − 0,5 durch beliebige nicht-oxidierende Säuren gefunden, das dadurch gekennzeichnet ist, daß man die Reaktion in Gegenwart von 0,02 bis 3 Mol Eisenionen pro Mol 3-Oxo-4-hydroxyimino-1,2,3,4-tetrahydronaphthalin-1-sulfonsäure durchführt.

3-Oxo-4-hydroxyimino-1,2,3,4-tetrahydronaphthalin-1-sulfonsäure (Hydrogensulfit-Additionsverbindung) kann nach dem Stand der Technik beispielsweise durch Nitrosierung von β-Naphthol und anschließende Addition von Alkalimetall-Hydrogensulfit erhalten werden. Man erhält hierbei diese Additionsverbindung in Form ihres Alkalimetallsalzes, beispielsweise des Natrium- oder Kaliumsalzes, durch Aussalzen, beispielsweise mit Natriumchlorid, oder die freie Säure durch anschließende Säurebehandlung eines solchen Alkalimetallsalzes. Erfindungsgemäß können sowohl die Säure als auch deren Alkalimetallsalze eingesetzt werden. Weiterhin kann sowohl eine wäßrige Lösung der zuvor isolierten 3-Oxo-4-hydroxyimino-1,2,3,4-tetrahydronaphthalin-1-sulfonsäure oder ihres Alkalimetallsalzes eingesetzt werden als auch wäßrige Reaktionsgemische, die durch Umsetzung von nitrosierten β-Naphthol mit Hydrogensulfit entstanden sind.

Erfindungsgemäß wird die Herstellung der Amidolsäure in Gegenwart von Eisenionen durchgeführt. Die Eisenionen können dem Reaktionsgemisch in Form von Eisenverbindungen zugesetzt werden, aus denen im Reaktionsgemisch Eisenionen gebildet werden können. Als solche Verbindungen seien beispielsweise Salze, Hydroxide, lösliche Oxide oder Komplexverbindungen des Eisens genannt. Als Salze seien beispielsweise die Sulfate, Nitrate, Chloride, Bromide, Jodide, Carbonate, Formiate, Acetate, Phosphate, genannt. Als Hydroxide und lösliche Oxide seien beispielsweise $Fe(OH)_2$, $Fe(OH)_3$, FeO oder $Fe_2O_3$ genannt. Die genannten Eisenverbindungen können sowohl einzeln als auch als Gemisch mehrerer von ihnen eingesetzt werden. Weiterhin können die genannten Eisenverbindungen in fester Form oder als eine Lösung in Wasser oder in nichtoxidierenden Säuren zugegeben werden.

Als Eisenionen für das erfindungsgemäße Verfahren seien ferner dreiwertige oder zweiwertige Eisenionen oder ein Gemisch aus dreiwertigen und zweiwertigen Eisenionen genannt. Bevorzugt werden erfindungsgemäß zweiwertige Eisenionen oder ein Gemisch, das überwiegend zweiwertige Eisenionen enthält, eingesetzt. Solche Gemische liegen in vielen technisch verfügbaren Salzen des zweiwertigen Eisens vor, die infolge ihres Kontaktes mit dem Luftsauerstoff einen geringen Anteil an Salz des dreiwertigen Eisens aufweisen.

Das Eisen wird in einer Menge von 0,02 Mol bis 3 Mol Eisenionen pro Mol Hydrogensulfit-Additionsverbindung eingesetzt, bevorzugt werden 0,05 bis 2 Mol, besonders bevorzugt 0,1 bis 1 Mol, Eisenionen eingesetzt. Größere Mengen an Eisen sind unschädlich, aber ohne besonderen Vorteil.

Das erfindungsgemäße Verfahren wird unter Ansäuern des Reaktionsansatzes durchgeführt. Dieses Ansäuern auf pH-Werte von etwa 1,5 bis minus 0,5 kann durch beliebige nicht-oxidierende Säuren geschehen. Genannt seien beispielsweise Schwefelsäure, Phosphorsäure oder Halogenwasserstoffsäuren, bevorzugt Schwefelsäure.

In einer bevorzugten Variante wird eine eisenhaltige Abfall-Dünnsäure eingesetzt, wie sie beispielsweise bei der Titandioxid-Herstellung anfällt. Eine solche Abfallschwefelsäure enthält gleichzeitig die erfindungsgemäß erforderlichen Eisenionen und die zur Ansäuerung erforderliche Schwefelsäure. Typische Abfallschwefelsäuren aus der Titandioxid-Herstellung enthalten etwa 20 bis 25 Gew.-% $H_2SO_4$ und etwa 10 bis 13 Gew.-% $FeSO_4$.

Das Reaktionsgemisch enthält weiterhin mindestens 1 Mol zusätzliches Hydrogensulfit pro Mol

2

Hydrogensulfit-Additionsverbindung. Durch Erwärmen des mit Hydrogensulfit und der Säure versetzten Reaktionsgemisches auf 10 bis 120 °C, bevorzugt 30 bis 80 °C, wird die Bildung der Amidolsäure, die bereits während der Ansäuerung einsetzt, vervollständigt.

Im Vergleich zu Verfahren ohne Zusatz von Eisenionen werden erfindungsgemäß beträchtliche Ausbeutesteigerungen erzielt. Der Einsatz von Eisenionen ist weiterhin wirtschaftlich wegen der beträchtlichen Erhöhung der Raum/Zeit-Ausbeute gegenüber dem Stand der Technik und gleichzeitig ökologisch unbedenklich. Bei der speziellen Verfahrensvariante der Verwendung von Abfall-Dünnsäure aus der Titandioxid-Herstellung steht zudem eine preisgünstige Schwefelsäure zur Verfügung, die die erforderlichen Mengen Eisensulfat bereits enthält und die dabei gleichzeitig eine sinnvolle Nutzung findet.

## Beispiel 1

1 Mol isolierte und umkristallisierte Bisulfit-Additionsverbindung (3-Oxo-4-hydroxyimino-1,2,3,4-tetrahydronaphthalin-1-sulfonsäure) in Form ihres Natriumsalzes wird in 2 500 ml Wasser gelöst und mit 1,5 Mol Natriumbisulfit als 38 gew.-%ige wäßrige Lösung und 0,8 Mol Eisen-II-sulfat versetzt. Die dunkle Reaktionslösung wird mit 50 %iger Schwefelsäure bei 25 °C auf pH 0,5 gestellt, wobei die Bildung der Amidolsäure langsam einsetzt. Durch 5-stündiges Erwärmen auf 50 °C wird die Reaktion vervollständigt. Anschließend wird die Suspension auf 20 °C abgekühlt, die ausgefallene Amidolsäure abfiltriert und gewaschen. Es werden 90 % der theoretischen Ausbeute, bezogen auf die Bisulfit-Additionsverbindung, erhalten.

## Beispiel 2

2 900 ml einer wäßrigen Lösung der Bisulfit-Additionsverbindung, hergestellt durch Nitrosierung von 1 Mol β-Naphthol und Addition von Bisulfit durch Zugabe von 2,5 Mol Natriumbisulfit als 38 gew.-%ige wäßrige Lösung (s. BIOS Final Report loc. cit.) werden mit 0,6 Mol Eisen-II-sulfat versetzt und anschließend mit 600 ml 70 %iger Schwefelsäure angesäuert. Bereits während der Ansäuerung beginnt Amidolsäure auszufallen. Durch Erwärmen auf 50 °C während 5 h wird die Reaktion vervollständigt. Nach Abkühlen auf 20 °C, Isolierung und Waschen des Produktes werden 86 % der theoretischen Ausbeute Amidolsäure, bezogen auf β-Naphthol, erhalten.

## Beispiel 3

Eine wie in Beispiel 2 hergestellte Lösung der Bisulfit-Additionsverbindung wird mit 2 500 g Abfalldünnsäure aus der Titandioxid-Herstellung (22-23 Gew.-% $H_2SO_4$, 11-11,5 Gew.-% $FeSO_4$) angesäuert. Die weitere Vorgehensweise entspricht Beispiel 2. Es werden 86,5 % der theoretischen Ausbeute, bezogen auf β-Naphthol, erhalten.

## Beispiel 4

(Vergleichsversuch nach BIOS Final Report No. 986/I, Item No. 22, S. 44-45 ; vgl. auch Ullmann, 4. Auflage, Band 17, Seite 100, 125).

1 Mol β-Naphthol wird nach Vorschrift nitrosiert und anschließend mit Bisulfit umgesetzt. Die so erhaltene Lösung der Bisulfit-Additionsverbindung, die geringe Mengen schwarzer Flocken enthält, wird mit 125 g Kochsalz versetzt und nach dessen Auflösung auf 1 250 ml 5,5 %ige Schwefelsäure gegossen. Durch weitere Zugabe von 175 g 78 %iger $H_2SO_4$ wird das Reaktionsgemisch noch stärker angesäuert und anschließend von außen auf 40 °C erwärmt. Zunächst steigt die Temperatur noch auf ca. 50 °C, um dann langsam abzufallen. Dabei fällt die Amidolsäure als hellgraues Produkt aus. Zur Vervollständigung der Kristallisation läßt man, unterbrochen von zwischenzeitlichem Durchrühren, noch 40 h stehen. Nach Isolierung und Waschen des Produktes werden 79 % der theoretischen Ausbeute, bezogen auf β-Naphthol, erhalten.

## Beispiel 5

(Vergleichsversuch nach Organic Synthesis Coll. Vol. I, S. 411 (1941) und Coll. Vol. II, S. 42 (1943))

2,1 Mol β-Naphthol werden nach Vorschrift nitrosiert, isoliert und zum Bisulfit-Additionsprodukt umgesetzt. Nach Entfernen der dunklen Flocken durch Klärung erhält man eine gelbbraune klare Lösung, die auf 7 l verdünnt wird. Unter kräftigem Rühren wird mit 400 ml konz. Schwefelsäure angesäuert. Dabei steigt die Temperatur des Reaktionsgemisches im Laufe von 2 h auf 50 °C. Nach weiteren 3 h wird das Produkt isoliert und gewaschen. Es werden 75 % der theoretischen Ausbeute, bezogen auf β-Naphthol, 100 %ige Amidolsäure erhalten.

Beispiel 6

Ansatz und Durchführung jeweils analog den Beispielen 1 und 2, jedoch ohne Zusatz des Eisensalzes.

| Vergleichsversuch zu | Ausbeute |
| --- | --- |
| Beispiel 1 | 81 %, bez. auf Bisulfit-Additionsverbindung |
| Beispiel 2 | 79 %, bez. auf $\beta$-Naphthol |

**Patentansprüche**

1. Verfahren zur Herstellung von 1-Amino-2-naphthol-4-sulfonsäure (Amidolsäure) durch Ansäuern von 3-Oxo-4-hydroxyimino-1,2,3,4-tetrahydronaphthalin-1-sulfonsäure oder ihres Alkalisalzes in Gegenwart von Hydrogensulfit auf pH-Werte von etwa 1,5 bis − 0,5 durch beliebige nicht-oxydierende Säuren, dadurch gekennzeichnet, daß man die Reaktion in Gegenwart von 0,02 bis 3 Mol Eisenionen pro Mol 3-Oxo-4-hydroxyimino-1,2,3,4-tetrahydronaphthalin-1-sulfonsäure durchführt.

2. Verfahren nach Anspruch 1, dadurch gekennzeichnet, daß man in Gegenwart von 2-wertigen Eisenionen arbeitet.

3. Verfahren nach Ansprüchen 1 bis 2, dadurch gekennzeichnet, daß man 0,05 bis 2 Mol Eisenionen pro Mol 3-Oxo-4-hydroxyimino-1,2,3,4-tetrahydronaphthalin-1-sulfonsäure einsetzt.

4. Verfahren nach Ansprüchen 1 bis 2, dadurch gekennzeichnet, daß man 0,1 bis 1 Mol Eisenionen pro Mol 3-Oxo-4-hydroxyimino-1,2,3,4-tetrahydronaphthalin-1-sulfonsäure einsetzt.

5. Verfahren nach Anspruch 1 bis 4, dadurch gekennzeichnet, daß man eine eisenhaltige Abfall-Schwefelsäure einsetzt.

**Claims**

1. Process for the preparation of 1-amino-2-naphthol-4-sulphonic acid (amidol acid) by acidifying 3-oxo-4-hydroxyimino-1,2,3,4-tetrahydronaphthalene-1-sulphonic acid or its alkali metal salt in the presence of hydrogen sulphite to pH values of about 1.5 to − 0.5 by means of any desired non-oxidising acids, characterised in that the reaction is carried out in the presence of 0.02 to 3 mols of iron ions per mol of 3-oxo-4-hydroxyimino-1,2,3,4-tetrahydronaphthalene-1-sulphonic acid.

2. Process according to Claim 1, characterised in that the reaction is carried out in the presence of 2-valent iron ions.

3. Process according to Claims 1 to 2, characterised in that 0.05 to 2 mols of iron ions are used per mol of 3-oxo-4-hydroxyimino-1,2,3,4-tetrahydronaphthalene-1-sulphonic acid.

4. Process according to Claims 1 to 2, characterised in that 0.1 to 1 mol of iron ions are used per mol of 3-oxo-4-hydroxyimino-1,2,3,4-tetrahydronaphthalene-1-sulphonic acid.

5. Process according to Claim 1 to 4, characterised in that an iron-containing waste sulphuric acid is used.

**Revendications**

1. Procédé de production d'acide 1-amino-2-naphtol-4-sulfonique (acide amidolique) par acidification d'acide 3-oxo-4-hydroxyimino-1,2,3,4-tétrahydronaphtalène-1-sulfonique ou de son sel alcalin en présence d'hydrogénosulfite à des valeurs de pH d'environ 1,5 à − 0,5 par des acides non oxydants quelconques, caractérisé en ce qu'on conduit la réaction en présence de 0,02 à 3 moles d'ions fer par mole d'acide 3-oxo-4-hydroxyimino-1,2,3,4-tétrahydronaphtalène-1-sulfonique.

2. Procédé suivant la revendication 1, caractérisé en ce qu'on opère en présence d'ions fer divalents.

3. Procédé suivant les revendications 1 et 2, caractérisé en ce qu'on utilise 0,05 à 2 moles d'ions fer par mole d'acide 3-oxo-4-hydroxyimino-1,2,3,4-tétrahydronaphtalène-1-sulfonique.

4. Procédé suivant les revendications 1 et 2, caractérisé en ce qu'on utilise 0,1 à 1 mole d'ions fer par mole d'acide 3-oxo-4-hydroxyimino-1,2,3,4-tétrahydronaphtalène-1-sulfonique.

5. Procédé suivant les revendications 1 à 4, caractérisé en ce qu'on utilise un acide sulfurique résiduaire contenant du fer.